# EUROPEAN PATENT SPECIFICATION

(11) **EP 0 389 561 B1**
(45) Date of publication and mention of the grant of the patent: **11.10.1995**
(21) Application number: 89901348.6
(22) Date of filing: 07.11.1988
(51) Int. Cl.: B32B 27/06, B32B 5/18, A61F 13/00, A41D 31/02

(54) **BREATHABLE NON-LINTING LAMINATE**
ATEMBARER, FUSSELFREIER VERBUNDSTOFF
LAMINE RESPIRABLE ET NE S'EFFILOCHANT PAS

(30) Priority: 13.11.1987 US 120330
(43) Date of publication of application: 03.10.1990
(73) Proprietor: W.L. GORE & ASSOCIATES, INC., Newark, Delaware 19714-9206 (US)
(72) Inventor: SAVILLE, Mark, Kendall, Newark, DE 19713 (US); GORAK, William, J., Jr., Elkton, MD 21921 (US); BOVE, Ronald, L., Newark, DE 19711 (US); KELLER, Mary, Lou, Newark, DE 19713 (US); BROWN, Peter, B., Elkton, MD 21921 (US)
(74) Representative: Klunker . Schmitt-Nilson . Hirsch
(86) International application number: PCT/US88/03997
(87) International publication number: WO 89/04253

(56) References cited:
- EP-A- 0 123 965
- EP-A- 0 160 473
- DE-A- 2 737 756
- US-A- 4 433 026
- US-A- 4 532 316

## Description

### FIELD OF THE INVENTION

The present invention relates to a flexible, breathable, non-linting, clean and cleanable composite laminate. Particularly, the invention relates to a composite laminate of a padding between two layers of water impermeable, moisture-vapor-permeable porous film, preferably porous, expanded polytetrafluoroethylene, useful, inter alia, as a covering for cleaned aerospace equipment which will keep such equipment absolutely clean during retrofitting, shipping and storage, as the padding underneath orthopedic casts and braces and as thermal insulation in wearing apparel. The layers of porous film surrounding the padding protect the padding, especially in applications where the padding is ordinarily susceptible to becoming wet.

### BACKGROUND OF THE INVENTION

For covering aerospace equipment, a clean and cleanable product is required which is non-linting and which is a very effective particle filter. The material must be sewable or joinable, it must dissipate electrical charges, and it must be non-flammable.

Aerospace equipment, especially equipment associated with shuttle and rocket flights, is assembled and/or cleaned in clean areas. Once cleaned, it is imperative to keep such equipment clean prior to and during use.

Equipment for use on shuttle flights needs to be kept free of dust, dirt and organic vapors. During retrofitting of the shuttle between flights, major pieces of equipment, e.g., the cargo doors, require such protection.

Heretofore, "Chemstat 919", a monofilament polyamide fabric sold under the trademark Nomex®, and having interwoven conducting thread, has been used, but this material is a poor particulate filter and can allow submicron particles through onto the equipment to be protected. Also, "Herculite", a plasticized PVC coating on nylon fabric has been used for clean area barriers, but can give off a plasticizer as a gas which can fog lenses.

Until now, there has been no entirely satisfactory covering material for such sensitive equipment.

Fabrics used for underpadding for limb casts have included cotton, foams, synthetics such as polyester fiberfill and wool. The major functions of underpadding are cushioning, filling and thermal insulation. Medical uses for paddings include applying or relieving pressure and absorption of excess moisture. Most currently available underpaddings lose some of their functionality when immersed in water. Water can cause padding to compress or to shift due to weight gain. It also alters the insulating properties of most paddings and can cause extreme discomfort. In addition, a water-saturated underpadding is not useful for transfer of moisture away from the skin. This loss of function is especially critical when the padding is worn by a person for extended periods. Prolonged wetness next to skin causes skin breakdown and possible infection sites. It is therefore desirable to provide a padding which will not be adversely affected by exposure to water. It is also desirable to provide a padding that will allow the transfer of water vapors so that perspiration and other body fluids can escape.

In the past, several methods have been used to overcome some of the drawbacks of padding. Quilting has been used to prevent padding from shifting, while maintaining high loft and bulkiness desirable in padding. Bonding padding to a fabric backing also prevents shifting. Fiberfill consists of crimped fibers to help maintain fluffiness and air space, even upon exposure to water. Hollow fibers also contain air space which add insulating value. Synthetic fibers are generally less absorbent than natural ones and have been used to wick fluids away from the skin into more absorbent backings. However, fluid saturation results in loss of function.

Large quantities of padding are used under immobilizing orthopedic casts or braces. Traditional cast underpadding consists of cotton or polyester wraps in 2 inch to 6 inch widths. Care is taken when applying the underpadding to avoid folds or creases which call cause pressure sores to the skin. The immobilizing material applied on top of this padding is usually made of plaster of paris or polyurethane-coated fiberglass. Cast wearers arc usually told not to immerse the cast in water, even though polyurethane casts can withstand water immersion. If a traditionally padded polyurethane cast is immersed, the padding remains wet for many hours, and the result can be skin maceration. In some cases, this maceration can lead to skin breakdown and infection by bacteria or fungi. Cast padding material manufacturers often caution against water immersion and state that if a cast is wet, it should be completely dried with a hair dryer. This drying process can take several hours and patient compliance is extremely low. Some cast wearers use plastic bags with rubber bands around the end to keep water away from the cast while showering. Water often enters the bag and wets the padding which causes discomfort to the wearer. As a result, many cast wearers do not want to get their cast wet and spend the time they are wearing a cast without a normal shower or bath. In addition, cast wearers cannot swim or use any form of hydrotherapy.

It is widely recognized that paddings must be "breathable" to be comfortable. However, it is not necessary that air pass through the padding for it to be comfortable, only that water vapor from perspiration or other sources be transmitted from the skin outwards through the padding. "Breathability" and the ability to transport interior moisture vapor to the external environment are used interchangeably herein.

As used herein, the term "porous, expanded polytetrafluoroethylene" is as disclosed in U.S. Patent 3,953,566. The term "breathable" as used herein means the ability to transport moisture from the humid side of a hydrophilic member and discharge the moisture on the dry side of the member, as disclosed in U.S. Patent 4,194,041. Both of these patents are assigned to assignee herein and both are incorporated here by reference thereto. The terms quilt, quilted and quilting refer to a pattern of discretely-shaped cells.

DE-A-2 737 756 discloses "laminates", i.e. composite materials comprising several layers being bonded together by a suitable adhesive or by applying heat and pressure. Contrary thereto the invention is a composite material comprising several layers where the layers are bonded together only along lines resulting in a quilt like pattern.

### SUMMARY OF THE INVENTION

According to the present invention, a flexible, breathable, non-linting, clean and cleanable composite laminate is provided, characterized by
(i) a middle layer of padding, and
(ii) a top and bottom layer each comprising waterimpermeable and moisture-vapor-permeable porous film, said top and bottom layers being bonded to said middle layer and defining discrete quilt patterns in which the middle layer and top and bottom layers are compressed and bonded along lines being the peripheral boundaries of the quilt pattern.

The preferred external surfaces of the composite laminate are films of porous, expanded polytetrafluoroethylene. The top and bottom layer each preferably comprise a flexible, first member of hydrophobic material having a moisture vapor transmission rate exceeding 1000 gms./m² · day. A continuous hydrophilic member may be joined with, i.e., attached to or penetrate the pores of the first member, the hydrophilic member having a moisture vapor transmission rate exceeding 1000 gms./m² · day and forming a barrier to liquids. The middle layer may be non-flammable and may contain an antistatic agent. The middle layer may be a synthetic material such as polyamide, polyester or polybenzimidazole fabric, woven, knitted or non-woven. It may comprise natural fabrics such as cellulostics or wool, or may be made of down. The middle layer may alternatively be a foam, a glass cloth, glass mat or any fabric of organic or inorganic fiber. Cationic, non-ionic or anionic antistatic agents may be used. Static electricity can also be dissipated with an electrically conducting material incorporated in the fabric. The bonding material may be a cured polyurethane based on the reaction product of (i) a polyol (A) having a number average molecular weight of from 600 to 3500 and having a functionality of at least 2; (ii) an isocyanate (B) having a functionality of at least 2; and (iii) a low molecular weight chain extender (C) having a molecular weight lower than 500 and having a functionality of at least 2, wherein the reactants are employed in such proportions so as to satisfy the following equations: 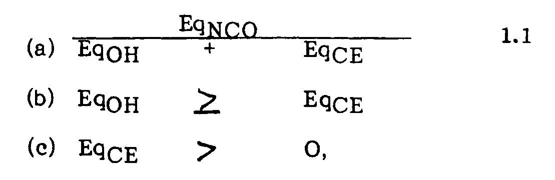
wherein Eq_{NCO} denotes the equivalents of the isocyanate species employed, and Eq_{OH} and Eq_{CE} denote the respective molar equivalents of the polyol and chain extender employed, characterized in that the reactants are selected such that the reaction product of the isocyanate and chain extender provides suitable hard segments and soft segments. Alternatively, the adhesive may be the hydrophilic member which is thermoplastic and is capable of bonding to itself or to other materials. Similarly, if the padding is a thermoplastic, it too can be capable of bonding the two outside layers together under the influence of heat.

The term "film" is used to include coatings formed by casting, or the like.

### BRIEF DESCRIPTION OF THE DRAWINGS

Fig. 1 is a perspective view, partly in cross-section, of the composite laminate according to the invention.

Fig. 2 is a top plan view, partly broken away, of the composite laminate of the invention.

Fig. 3 is a top plan view of one embodiment of a quilted composite according to the invention.

Fig. 4 is a top plan view of an alternate embodiment of a quilted composite according to the invention.

Fig. 5 is a cross-sectional view taken along line 5-5 of Fig. 4.

### DETAILED DESCRIPTION OF THE INVENTION AND PREFERRED EMBODIMENTS WITH REFERENCE TO THE DRAWINGS

A flexible, breathable, non-linting, clean and cleanable composite laminate which may be antistatic and/or non-flammable is provided. The laminate comprises a middle layer of a padding preferably a polyamide, polyester or glass fabric, and preferably being non-flammable and containing an antistatic agent when used in aerospace applications; and a top and bottom layer of water impermeable and moisture-vapor-permeable film. Preferably a film of porous, expanded polytetrafluoroethylene is bonded to the middle layer by means of an adhesive between the middle layer and the top and bottom layers. Preferably the top and bottom layers comprise a flexible, first member of hydrophobic material having a moisture vapor transmission rate exceeding 1000 gms./m² · day. A continuous hydrophilic member may be joined with the first member, the the hydrophilic member having a moisture vapor transmission rate exceeding 1000 gms./m² · day and forming a barrier to passage of liquids where the hydrophobic members are both external to the middle layer.

The laminate is useful in diverse applications such as a cover for cleaned aerospace equipment during retrofitting, shipping and storage, as a padded liner under orthopedic casts and braces and as an insulation in wearing apparel.

A detailed description of the invention and preferred embodiments is best provided with reference to the drawings.

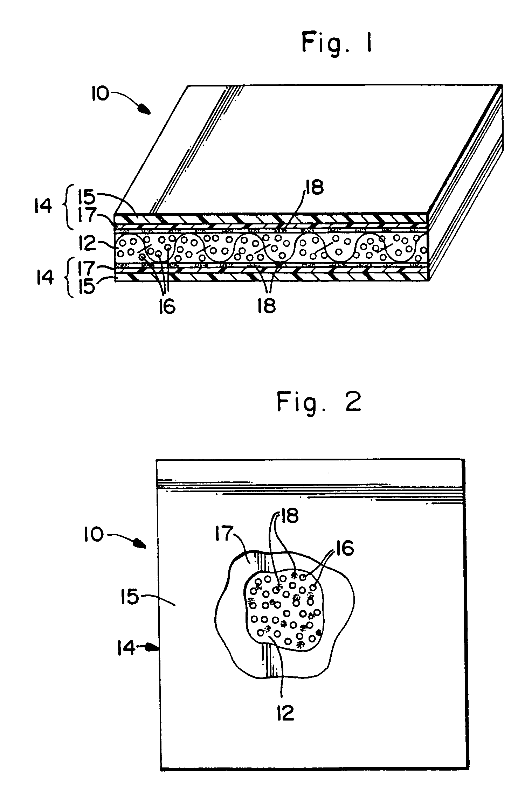

Although useful for understanding the invention, the composite laminates described with reference to Figures 1 and 2 and the composite laminates described in Examples 1 and 2 are not embodiments of the invention.

Fig. 1 is a perspective view, partly in cross-section, of a composite laminate 10. Laminate 10 includes middle layer 12 of a padding which may contain an antistatic agent 16. The middle layer 12 preferably is a non-flammable polyamide, woven, knitted or non-woven, marketed by DuPont under the trademark NOMEX® when used in aerospace applications and polyester when used in casts. An antistatic agent which is suitable is Zelec® DP, marketed by DuPont, and described to be a dispersion of complex cationic polymers. Bonded to each side of middle layer 12 is a layer 14 containing a first member 15 of water impermeable, moisture-vapor-permeable film, preferably expanded, porous polytetrafluoroethylene. Layers 14 are bonded to layer 12. One means of bonding can be an adhesive 18 as shown between the middle layer 12 and the top and bottom layers 14. Layers 14 as shown comprise the hydrophobic members 15 and the breathable hydrophilic members 17 as disclosed in U.S. Patent 4,194,041, and this is preferred for certain uses. However, the members 15 may be bonded directly to the middle layer 12 by adhesive 18. In some cases, the hydrophobic member forms the external surfaces of the composite laminate 10. For aerospace applications, the adhesive 18 may be the material disclosed in U.S. Patent 4,532,316; namely, a cured polyurethane based on the reaction product of (i) a polyol (A) having a number average molecular weight of from 600 to 3500 and having a functionality of at least 2; (ii) an isocyanate (B) having a functionality of at least 2; and (iii) a low molecular weight chain extender (C) having a molecular weight lower than 500 and having a functionality of at least 2, wherein the reactants are employed in such proportions so as to satisfy the following equations: 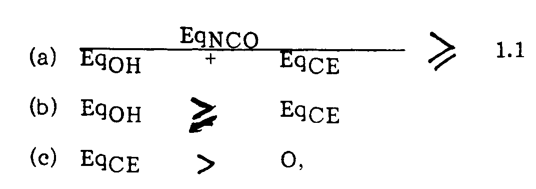
wherein Eq_{NCO} denotes the equivalents of the isocyanate species employed, and Eq_{OH} and Eq_{CE} denote the respective molar equivalents of the polyol and chain extender employed, characterized in that the reactants are selected such that the reaction product of the isocyanate and chain extender provides suitable hard segments and soft segments.

Fig. 2 shows a top plan view of a composite laminate 10 for use in coverings for aerospace equipment, partially broken away. Therein, middle layer 12 contains antistatic agent 16 and is bonded to top layer 14 by adhesive 18. Layer 14 is shown comprised of inner hydrophilic member 17 and outer, or external layer 15 of porous, water permeable, moisture-vapor-permeable film.

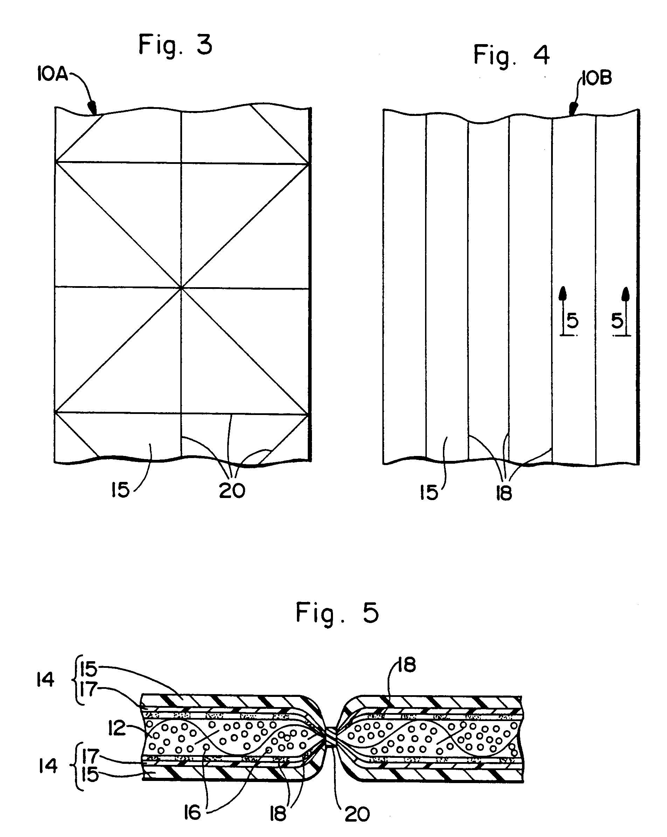

Fig. 3 is a top plan view of a quilted embodiment 10A of padded liner for use in an orthopedic cast according to the invention, wherein the quilted pattern is in the form of triangles bounded by quilt lines 20. Fig. 4 is an alternate embodiment 10B having parallel quilt lines 20, and Fig. 5 shows a cross-sectional view taken along line 5-5 of Fig. 4.

For use as a padded liner under a cast, it is desirable to divide the composite laminate into smaller units or cells through quilting or embossing. This is useful not only to prevent shifting but also to protect each cell from water which may enter through a hole or defect in adjacent cells. This will allow the quilted composite laminate to be cut or trimmed without violating the integrity of the whole pad.

The quilted composite laminate can be manufactured by sealing the edges of the cells to completely encompass the padding, as shown in Fig. 3. This sealing can be done in many ways, for example, using direct heat or ultrasonic welding or adhesives.

When the composite laminate is used as a padded liner in a cast, the outer layers do not allow liquid water to pass through to the inner layers. However, water will pass through the film after it has been vaporized. Additionally, the body heat of the wearer causes this evaporation process to occur and creates a one-way path for water vapor. If the padding becomes moist, the moisture is still directed outwardly through the cast, not back towards the person. Therefore, padding encapsulated in quilted composite laminates of this invention does not experience the same water weight gain that unprotected underpadding does, and the wearer does not have to wait as long to experience dryness. The skin surface dries much faster with the breathable composite laminates of this invention than with unprotected underpadding. Perspiration is allowed to pass through the breathable composite laminates without moisture remaining on the skin. Traditional underpadding remains damp for many hours, but the padding in composite laminates of this invention dries quickly. This provides the wearer with a great deal of comfort.

A number of commercially available materials are suitable for use as the water impermeable, moisture-vapor-permeable, porous film. These materials include microporous expanded polytetrafluoroethylene as described in U.S. Patents 3,953,566 and 4,187,390; and expanded polytetrafluoroethylene coated with hydrophilic impregnants and layers, such as described in U.S. Patent 4,194,041. Alternatively, microporous polyolefin films or scrims which may be coated with or impregnated with hydrophilic polymers such as certain polyurethanes are also suitable for this use. The term "polyolefin" as used herein includes both halogen-free and halogenated polyolefins, e.g., polypropylene or polytetrafluoroethylene.

There are many benefits of using a waterproof, breathable orthopedic cast padded liner. The products of this invention allow cast or brace wearers to shower or swim while being immobilized without special precautions or drying procedures. This is advantageous not only for hygenic purposes, but also for various therapeutic reasons. Odor and itching can be reduced for cast patients if washing is allowed on a normal schedule. Cast wearers can engage in activities which may cause perfuse perspiration without the discomfort of wet underpadding. Use of hydrotherapy could aid in the healing process, as it does for some other injuries which do not require immobilization.

The breathable composite laminates of this invention can be applied in a wrap formation similar to traditional cast underpaddings, or they can be configured to fit around the damaged limb in a single layer. The thinness of the film is essential to avoid bulky folds which can cause pressure sores on the skin.

Many athletes require the use of supportive braces to continue their training. Use of this padding would prevent perspiration from being trapped next to the skin following exertion. Alternatively, this padding could be incorporated directly into the brace.

The breathable quilted composite laminates are also useful as thermal insulation in wearing apparel.

### WATER VAPOR TRANSMISSION RATE

Water vapor transmission rate was determined by ASTM 96-8 inverted cup method at 23° ± 1°C., at 50% relative humidity with a wind velocity of 500-600 FPM.

### EXAMPLE 1

A composite laminate was made by the following procedure. Dilutions of Zelec® DP antistatic agent, marketed by DuPont, of 5.0%, 2.5% and 1.0% were made. A fabric of Nomex® polyamide fibers was first bonded to a two-component sheet of expanded, porous polytetrafluoroethylene and a hydrophilic member as described in U.S. Patent 4,194,041 with the porous, polytetrafluoroethylene portion positioned externally, using an adhesive as described in U.S. Patent 4,532,316 containing Antiblaze® 100 flame retardant, marketed by Albright Wilson, Inc. The dilutions of antistatic agent described above were padded into three specimens of this Nomex® fabric, partially air-dried and then heated to 140°C in a convection oven. These samples were then laminated on the Nomex® side to a second 2-component sheet of expanded, porous polytetrafluoroethylene and a hydrophilic member as described above, again with the adhesive between the inner and outer portions. In this example, the expanded, porous polytetrafluoroethylene formed the external surfaces of the composite.

The specimens were tested by the NFPA 99 Static Decay Test, which measures the time of decay from a charge of 5000 volts to 500 volts, in seconds. Results are shown in Table 1.

**Table 1**

| Sample 1 | % Zelec® DP | Dry Pick-Up of Antistat, Wt.% of Middle and Top Layers | Time of Decay of Laminate, sec. |
|---|---|---|---|
| 1 | 5% | 0.39 | .08-.09 |
| 2 | 2.5% | 0.17 | .12-.32 |
| 3 | 1% | 0.04 | 1.90-2.53 |
| 4 (Control) | 0% | 0 | 20 |

A flammability test was performed on the same samples as above according to the Federal Test standard number 191A, Method 5903. Results are shown in Table 2.

**Table 2**

| Sample 2 | After flame, seconds | After glow, seconds | Charred length,cm (inches) |
|---|---|---|---|
| 1 | 0 | 0.9 | 6,6 (2,6) |
| 2 | 0 | 1.4 | 4,06 (1,6) |
| 3 | 0 | 1.5 | 4,83 (1,9) |
| 4 (Control) | 0 | 1.6 | 4,83 (1,9) |

### EXAMPLE 2

A porous, expanded polytetrafluoroethylene sheet was joined with a hydrophilic polymer, 22,86 cm (9 inches) wide and 3,66 m (4 yards) long, as disclosed in U.S. Patent 4,194,041, was laid down on a flat rubber mat, hydrophilic side up. A 10,16 cm (4 inch) wide strip of polyester padding was laid on top. The two-member sheet was folded over the padding. A sealing iron at approximately 270°C was used to make a seam down the open edge, enclosing the padding within the two-member sheet. The excess two-member sheet was trimmed, excess air was voided, and the ends were sealed. The resultant padded finer was worn under a polyurethane-coated fiberglass cast. The wearer showered daily, swam and used hydrotherapy on several occasions. No skin maceration was noted after an extended trial. No water leakage occured into the padding.

While the invention has been disclosed herein in connection with certain embodiments and detailed descriptions, it will be clear to one skilled in the art that modifications or variations of such details can be made without deviating from the gist of this invention, and such modifications or variations are considered to be within the scope of the claims hereinbelow.

## Claims

1. A flexible, breathable, non-linting, clean and cleanable composite laminate comprising:
(i) a middle layer of padding, and
(ii) a top and bottom layer each comprising waterimpermeable and moisture-vapor-permeable porous film, said top and bottom layers being bonded to said middle layer and defining discrete quilt patterns in which the middle layer and top and bottom layers are compressed and bonded along lines being the peripheral boundaries of the quilt pattern.

2. The composite laminate of claim 1 wherein said top and bottom layers each comprise:
(i) a flexible, water-impermeable, moisture-vapor-permeable, porous film as the external surfaces, and
(ii) a continuous hydrophilic layer joined to the film said hydrophilic layer having a moisture vapor transmission rate exceeding 1000 gms./m² · day and forming a barrier to passage of liquids wherein the layer (ii) is adjacent to said padding.

3. The composite laminate of claim 1 wherein the top and bottom layers each comprises:
(i) a flexible, water-impermeable, moisture-vapor-permeable porous film, and
(ii) a continuous hydrophilic layer joined to the film, said hydrophilic layer having a water vapor transmission rate exceeding 1000 gms./m² · day and forming a barrier to passage of liquids, wherein the layer (ii) is on the external sides of the top and bottom layers.

4. The composite laminate of claim 1 wherein the porous water impermeable, water-vapor-permeable film is porous, expanded polytetrafluoroethylene.

5. The composite laminate of claim 2 or 3 wherein the porous water-impermeable, moisture-vapor-permeable film is porous, expanded polytetrafluoroethylene and the hydrophilic layer is a polyurethane

6. The composite of claim 1 wherein said middle layer is a nonflammable fabric.

7. The composite of claim 1 wherein said middle layer contains an antistatic agent.

8. The composite of claim 1 wherein said middle layer is electrically conductive.

9. The composite of claim 1 wherein said middle layer is a polyamide.

10. The composite of claim 9 wherein said middle layer is a woven polyamide fabric.

11. The composite of claim 9 wherein said middle layer is a knitted polyamide fabric.

12. The composite of claim 9 wherein said middle layer is a non-woven polyamide fabric.

13. The composite of claim 1 wherein said middle layer is a glass cloth.

14. The composite of claim 1 wherein said middle layer is a glass mat.

15. The composite of claim 1 wherein said middle layer is cotton.

16. The composite of claim 1 wherein said middle layer is foam.

17. The composite of claim 1 wherein said middle layer is polyester.

18. The composite of claim 1 wherein said middle layer is wool.

19. The composite of claim 1 wherein said middle layer is polybenzimidazole.

20. The composite of claim 7 wherein said antistatic agent is a dispersion of cationic polymers.

21. The composite of claim 7 wherein said antistatic agent is an electrically conducting material.

22. The composite of claim 1 wherein said top and bottom layers are bonded by an adhesive that is a cured polyurethane comprising the reaction product of:
(i) a polyol (A) having a number average molecular weight of from 600 to 3500 and having a functionality of at least 2;
(ii) an isocyanate (B) having a functionality of at least 2; and
(iii) a low molecular weight chain extender (C) having a molecular weight lower than 500 and having a functionality of at least 2, wherein the reactants are employed in such proportions so as to satisfy the following equations: 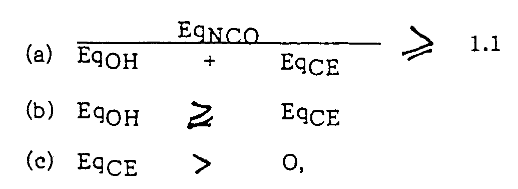 wherein Eq_{NCO} denotes the equivalents of the isocyanate species employed, and Eq_{OH} and Eq_{CE} denote the respective molar equivalents of the polyol and chain extender employed, characterized in that said reactants are selected such that the reaction product of the isocyanate and chain extender provides suitable hard segments and soft segments and wherein suitable hard segments are thermodynamically incompatible with said soft segments, the latter provided by the polyol, whereby phase separation of hard and soft segments in said prepolymer occurs at room temperature and renders said prepolymer a 100% solids, storage stable, segmented polyurethane prepolymer being an opaque solid at room temperature, said prepolymer having storage stability exceeding one month and being melt processible at room temperature below what would be considered to be the hard segment melt temperature.

23. Wearing apparel containing the composite laminate of claim 1.

24. Thermal insulation containing the composite laminate of claim 1.

25. An orthopedic immobilizer containing the composite laminate of claim 1.

## Patentansprüche

1. Flexibler, atmungsfähiger, nicht flusender, sauberer und reinigbarer zusammengesetzter Schichtstoff, umfassend:
(i) eine Mittelschicht aus einer Polsterung, und
(ii) eine obere und eine untere Schicht, jeweils umfassend eine wasserundurchlässige und für Feuchtigkeitsdampf durchlässige poröse Folie, wobei die obere und die untere Schicht mit der Mittelschicht verbunden sind und diskrete Steppmuster definieren, in denen die Mittelschicht und die oberen und die unteren Schichten entlang Linien zusammengedrückt und verbunden sind, welche die Umfangsgrenzen des gesteppten Musters bilden.

2. Schichtstoff nach Anspruch 1, bei dem die obere und die untere Schicht jeweils aufweisen:
(i) eine flexible, wasserundurchlässige, für Feuchtigkeitsdampf durchlässige poröse Folie als Außenflächen, und
(ii) eine durchgehende hydrophile Schicht, die mit der Folie verbunden ist, wobei die hydrophile Schicht eine Feuchtigkeitsdampfdurchlaßrate von mehr als 1000 g/m² · Tag aufweist und gegen das Durchdringen von Flüssigkeiten eine Barriere bildet, wobei die Schicht (ii) der Polsterung benachbart ist.

3. Schichtstoff nach Anspruch 1, bei dem die obere und die untere Schicht jeweils aufweisen:
(i) eine flexible, wasserundurchlässige, für Feuchtigkeitsdampf durchlässige poröse Folie, und
(ii) eine durchgehende hydrophile Schicht, die mit der Folie verbunden ist, wobei die hydrophile Schicht eine Wasserdampfdurchlaßrate von mehr als 1000 g/m² · Tag aufweist und gegen das Durchdringen von Flüssigkeiten eine Barriere bildet, wobei die Schicht (ii) sich auf den Außenseiten der oberen und der unteren Schichten befindet.

4. Schichtstoff nach Anspruch 1, bei dem die poröse, wasserundurchlässige und wasserdampfdurchlässige Folie poröses, expandiertes Polytetrafluorethylen ist.

5. Schichtstoff nach Anspruch 2 oder 3, bei dem die poröse wasserundurchlässige feuchtigkeitsdampfdurchlässige Folie porös ist, expandiertes Polytetrafluorethylen und die hydrophile Schicht ein Polyurethan ist.

6. Schichtstoff nach Anspruch 1, bei dem die Mittelschicht ein nichtentflammbarer Stoff ist.

7. Schichtstoff nach Anspruch 1, bei dem die Mittelschicht ein Antistatikmittel enthält.

8. Schichtstoff nach Anspruch 1, bei dem die Mittelschicht elektrisch leitend ist.

9. Schichtstoff nach Anspruch 1, bei dem die Mittelschicht ein Polyamid ist.

10. Schichtstoff nach Anspruch 9, bei dem die Mittelschicht ein gewobener Polyamidstoff ist.

11. Schichtstoff nach Anspruch 9, bei dem die Mittelschicht ein gestrickter Polyamidstoff ist.

12. Schichtstoff nach Anspruch 9, bei dem die Mittelschicht ein nichtgewebter Polyamidstoff ist.

13. Schichtstoff nach Anspruch 1, bei dem die Mittelschicht ein Glastuch ist.

14. Schichtstoff nach Anspruch 1, bei dem die Mittelschicht eine Glasmatte ist.

15. Schichtstoff nach Anspruch 1, bei dem die Mittelschicht Baumwolle ist.

16. Schichtstoff nach Anspruch 1, bei dem die Mittelschicht ein Schaum ist.

17. Schichtstoff nach Anspruch 1, bei dem die Mittelschicht Polyester ist.

18. Schichtstoff nach Anspruch 1, bei dem die Mittelschicht Wolle ist.

19. Schichtstoff nach Anspruch 1, bei dem die Mittelschicht Polybenzimidazol ist.

20. Schichtstoff nach Anspruch 7, bei dem das Antistatikmittel eine Dispersion von kationischen Polymeren ist.

21. Schichtstoff nach Anspruch 7, bei dem das Antistatikmittel ein elektrisch leitendes Material ist.

22. Schichtstoff nach Anspruch 1, bei dem die obere und die untere Schicht mit einem Klebstoff verbunden sind, bei dem es sich um ein ausgehärtetes Polyurethan handelt, umfassend das Reaktionsprodukt von:
(i) einem Polyol (A), dessen mittleres Molekulargewicht zwischen 600 und 3500 liegt, und das eine Funktionalität von mindestens 2 besitzt;
(ii) ein Isocyanat (B) mit einer Funktionalität von mindestens 2;
und
(iii) einem Niedermolekulargewicht-Kettenerweiterer (C) mit einem Molekulargewicht von unter 500 und mit einer Funktionalität von mindestens 2, wobei die Reaktionsmittel in solchen Anteilen eingesetzt werden, daß die folgenden Gleichungen erfüllt sind: 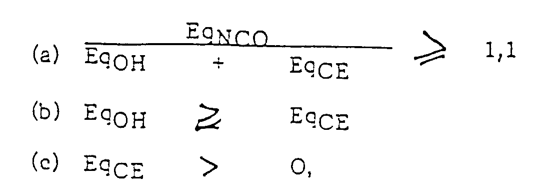 wobei Eq_{NCO} die Äquivalente der verwendeten Isocyanatspezies und Eq_{OH} und Eq_{CE} die jeweiligen molaren Äquivalente des Polyols und des verwendeten Kettenerweiterers bedeuten,
**dadurch gekennzeichnet,**
daß die Reaktionsmittel derart ausgewählt sind, daß das Reaktionsprodukt des Isocyanats und des Kettenerweiterers geeignete harte Segmente und weiche Segmente bildet, wobei geeignete harte Segmente thermodynamisch nicht mit den weichen Segmenten kompatibel sind, letztere durch das Polyol geschaffen werden, wodurch bei Zimmertemperatur eine Phasentrennung der harten und der weichen Segmente in dem Vorpolymer stattfindet und das Vorpolymer zu einem 100% festen, speicherstabilen, segmentierten Polyurethan-Vorpolymer macht, das bei Zimmertemperatur ein opaker Feststoff ist, wobei das Vorpolymer eine Speicherstabilität besitzt, die einen Monat übersteigt, und schmelzverarbeitbar bei Zimmertemperatur unterhalb dessen ist, was als Hartsegment-Schmelztemperatur verstanden wird.

23. Bekleidungsstück mit dem Schichtstoff nach Anspruch 1.

24. Wärmeisolierung mit dem Schichtstoff nach Anspruch 1.

25. Orthopädischer Ruhigsteller, beinhaltend den Schichtstoff nach Anspruch 1.

## Revendications

1. Stratifié composite propre et nettoyable, flexible, "qui respire" et ne peluche pas, comprenant :
(i) une couche moyenne de rembourrage et
(ii) une couche supérieure et une couche inférieure comprenant chacune un film poreux imperméable à l'eau et perméable à la vapeur d'humidité, lesdites couches supérieure et inférieure étant collées à ladite couche moyenne et définissant des dessins piqués discrets, dans lequel la couche moyenne et les couches supérieure et inférieure sont comprimées et collées le long de lignes constituant les frontières périphériques du dessin piqué.

2. Stratifié composite selon la revendication 1, dans lequel lesdites couches supérieure et inférieure comprennent chacune :
(i) un film poreux flexible, imperméable à l'eau, perméable à la vapeur d'humidité comme surfaces externes et
(ii) une couche hydrophile continue réunie au film, ladite couche hydrophile ayant une vitesse de transmission de la vapeur d'humidité supérieure à 1000 g/m² et par jour et formant une barrière au passage de liquides, la couche (ii) étant adjacente audit rembourrage.

3. Stratifié composite selon la revendication 1, dans lequel les couches supérieure et inférieure comprennent chacune :
(i) un film poreux flexible, imperméable à l'eau, perméable à la vapeur d'humidité et
ii) une couche hydrophile continue réunie au film, ladite couche hydrophile ayant une vitesse de transmission de la vapeur d'eau supérieure à 1000 g/m² et par jour et formant une barrière au passage de liquides, la couche (ii) se trouvant sur les faces externes des couches supérieure et inférieure.

4. Stratifié composite selon la revendication 1, dans lequel le film poreux imperméable à l'eau, perméable à la vapeur d'eau, est du polytétrafluoroéthylène poreux expansé.

5. Stratifié composite selon la revendication 2 ou 3, dans lequel le film poreux imperméable à l'eau, perméable à la vapeur d'humidité est du polytétrafluoroéthylène poreux expansé et la couche hydrophile est un polyuréthane.

6. Composite selon la revendication 1, dans lequel ladite couche moyenne est une étoffe non inflammable.

7. Composite selon la revendication 1, dans lequel ladite couche moyenne contient un agent antistatique.

8. Composite selon la revendication 1, dans lequel ladite couche moyenne est conductrice de l'électricité.

9. Composite selon la revendication 1, dans lequel ladite couche moyenne est un polyamide.

10. Composite selon la revendication 9, dans lequel ladite couche moyenne est une étoffe de polyamide tissée.

11. Composite selon la revendication 9, dans lequel ladite couche moyenne est une étoffe de polyamide tricotée.

12. Composite selon la revendication 9, dans lequel ladite couche moyenne est une étoffe de polyamide non tissée.

13. Composite selon la revendication 1, dans lequel ladite couche moyenne est un tissu de verre.

14. Composite selon la revendication 1, dans lequel ladite couche moyenne est un mat de verre.

15. Composite selon la revendication 1, dans lequel ladite couche moyenne est du coton.

16. Composite selon la revendication 1, dans lequel ladite couche moyenne est une mousse.

17. Composite selon la revendication 1, dans lequel ladite couche moyenne est du polyester.

18. Composite selon la revendication 1, dans lequel ladite couche moyenne est de la laine.

19. Composite selon la revendication 1, dans lequel ladite couche moyenne est du polybenzimidazole.

20. Composite selon la revendication 7, dans lequel ledit agent antistatique est une dispersion de polymères cationiques.

21. Composite selon la revendication 7, dans lequel ledit agent antistatique est un matériau conducteur de l'électricité.

22. Composite selon la revendication 1, dans lequel lesdites couches supérieure et inférieure sont collées par une colle qui est du polyuréthane durci, comprenant le produit de la réaction entre :
(i) un polyol (A) ayant une masse moléculaire moyenne en nombre comprise entre 600 et 3500 et une fonctionnalité d'au moins 2 ;
(ii) un isocyanate (B) ayant une fonctionnalité d'au moins 2 ; et
(iii) un extenseur de chaîne de masse moléculaire faible (C) ayant une masse moléculaire inférieure à 500 et une fonctionnalité d'au moins 2, les partenaires réactionnels étant employés dans des proportions aptes à satisfaire aux équations suivantes : 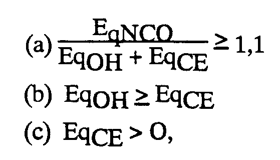 dans lesquelles Eq_{NCO} désigne les équivalents de l'espèce d'isocyanate utilisée et Eq_{OH} et Eq_{CE} désignent les équivalents molaires respectifs du polyol et de l'extenseur de chaîne utilisés, caractérisée en ce que lesdits partenaires réactionnels sont sélectionnés de façon que le produit réactionnel de l'isocyanate et de l'extenseur de chaîne fournisse des segments durs et des segments mous appropriés, et dans lequel les segments durs appropriés sont thermodynamiquement incompatibles avec lesdits segments mous, ces derniers étant fournis par le polyol, une séparation de phase des segments durs et mous dans ledit prépolymère survenant à la température ambiante et transformant ledit prépolymère en un solide à 100 % stable au stockage, le prépolymère de polyuréthane segmenté étant un solide opaque à la température ambiante, ledit prépolymère ayant une stabilité au stockage supérieure à un mois et étant traitable par fusion à une température ambiante inférieure à ce qui serait considéré comme la température de fusion des segments durs.

23. Vêtement contenant le stratifié composite selon la revendication 1.

24. Isolant thermique contenant le stratifié composite selon la revendication 1.

25. Moyen d'immobilisation orthopédique contenant le stratifié composite selon la revendication 1.
